(19) Europäisches Patentamt
European Patent Office
Office européen des brevets

(11) EP 2 986 974 B1

(12) EUROPEAN PATENT SPECIFICATION

(45) Date of publication and mention of the grant of the patent:
**06.05.2020 Bulletin 2020/19**

(21) Application number: **14715820.8**

(22) Date of filing: **19.03.2014**

(51) Int Cl.:
***G01N 23/12*** (2018.01) ***G01N 33/28*** (2006.01)
***G21G 4/06*** (2006.01) ***H05G 2/00*** (2006.01)

(86) International application number:
**PCT/US2014/031187**

(87) International publication number:
**WO 2014/153392 (25.09.2014 Gazette 2014/39)**

# (54) RADIATION SOURCE DEVICE

STRAHLENQUELLENVORRICHTUNG

DISPOSITIF SOURCE DE RAYONNEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.03.2013 US 201361803608 P**

(43) Date of publication of application:
**24.02.2016 Bulletin 2016/08**

(73) Proprietor: **Geoservices Equipements SAS**
**92140 Clamart (FR)**

(72) Inventors:
- **FIORE, Massimiliano**
  **92142 Clamart cedex (FR)**
- **CHAZAL, Damien**
  **F-67049 Roissy (FR)**
- **JOLIVET, Guillaume**
  **F-67049 Roissy (FR)**

(74) Representative: **Schlumberger Intellectual Property Department**
**Parkstraat 83**
**2514 JG Den Haag (NL)**

(56) References cited:
**EP-A1- 0 236 623**
**WO-A1-01/25762**
**DE-A1- 3 424 937**
**US-A- 3 655 984**
**US-A- 4 388 530**
**US-A- 4 450 576**
**US-A1- 2011 278 445**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 986 974 B1

Printed by Jouve, 75001 PARIS (FR)

## Description

The invention relates to a radiation source device and a measuring device using such a radiation source device for multiphase fluid analysis.

## BACKGROUND

### Description of the Related Art

X- and γ-ray radiation sources are used in a wide variety of applications. For example, they can be used to calibrate equipment, or used in energy-dispersive X-ray fluorescence (EDXRF) analyzers or in multiphase fluid flow analyzers.

A fluorescent X-ray is created when a photon of sufficient energy strikes an atom in the sample, dislodging an electron from one of the atom's inner orbital shells (lower quantum energy states). The atom regains stability, filling the vacancy left in the inner orbital shell with an electron from one of the atom's higher quantum energy orbital shells. The electron drops to the lower energy state by releasing a fluorescent X-ray, and the energy of this fluorescent X-ray (often measured in electron volts, eV) is equal to the specific difference in energy between two quantum states of the dropping electron. The high energy photons (X-rays or γ-rays) are provided by an X-ray or γ-particle source.

Presently, small X- and γ-ray sources often comprise a metal shell (e.g., stainless steel) with an open end into which a holder is inserted. The holder has a front face which carries the radiation source. The radiation source is a radioactive foil or other material. In front of the same foil, to seal off the open end of the metal shell is a radiolucent window, such as beryllium, which is brazed in place to seal it off.

In multiphase fluid analysis, photons interact with the multiphase fluid which absorbs a portion of the photons depending on the multiphase fluid composition. Initial emitted photons are absorbed, or attenuated, by the multiphase fluid and received by a detector- Attenuations are calculated by counting the photons of the specified energy levels impacting a detector after interacting with the multiphase fluid. Flow rates of three phases of the multiphase fluid may be obtained from phase fractions calculated using the attenuations. However, the number of X-rays and γ-rays emitted by a radioactive source in a time interval is not constant. Radioactive decay follows the Poisson statistical model, stating that the number of photons per second with energy $E$ $n_E$ averaged over a time interval $t$ is known with an uncertainty $\pm\sqrt{n_E/t}$. Therefore, attenuations and phase fractions are also affected by statistical uncertainties which can be reduced by increasing the acquisition time t. However, increasing acquisition time, from an operational point of view, reduces a number of tests able to be performed during a predetermined time period.

US4,388,530, US2011/0278445 and US4,450,576 disclose methods and apparatus for analysing fluids using Gamma ray and/or X-ray fluorescence analysis. DE3424937 discloses the use of X-ray fluorescence for measuring the concentration of a powder sample. US3,655,984 discloses a substantially immobile relatively low energy radiation source that includes a particulate radioisotope bearing material.

## SUMMARY

This summary is provided to introduce a selection of concepts that are further described in the detailed description. This summary is not intended to identify key or essential features of the claimed subject matter, nor is it intended to be used as an aid in limiting the scope of the claimed subject matter.

In a first aspect, a radiation source device according to independent claim 1 is described.

In an embodiment, a measuring device according to claim 6 is described, which comprises the radiation source device of the first aspect.

In a second aspect, a method according to independent claim 12 is described.

## BRIEF DESCRIPTION OF THE DRAWINGS

Certain embodiments of the present inventive concepts will hereafter be described with reference to the accompanying drawings, wherein like reference numerals denote like elements, and:

Figure 1 is a partial sectional view of a radiation source device in accordance with the present disclosure.

Figure 2 is a diagrammatic cross sectional view of one embodiment of the radiation source device of Figure 1.

Figure 3 is a diagrammatic cross sectional view of another embodiment of the radiation source device of Figure 1.

Figure 4 is a diagrammatic cross sectional view of yet another embodiment of the radiation source device of Figure 1.

Figure 5-1 is a diagrammatic representation of a measuring device using the radiation source device of Figure 1 in accordance with the present disclosure.

Figure 5-2 is a diagrammatic representation of an embodiment of a measuring device using the radiation source device of Figure 1 in accordance with the present disclosure.

Figure 6 is a diagrammatic representation of a method of using a measuring device with a radiation source device in accordance with the present disclosure.

Figure 7 is a graphical representation of the attenuation triangles generated using a measurement device with a radiation source device.

## DETAILED DESCRIPTION

Specific embodiments of the present disclosure will now be described in detail with reference to the accompanying drawings. Further, in the following detailed description of embodiments of the present disclosure, numerous specific details are set forth in order to provide a more thorough understanding of the disclosure. However, it will be apparent to one of ordinary skill in the art that the embodiments disclosed herein may be practiced without these specific details. In other instances, well-known features have not been described in detail to avoid unnecessarily complicating the description.

Unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the inventive concept. This description should be read to include one or at least one and the singular also includes the plural unless otherwise stated.

Finally, as used herein any references to "one embodiment" or "an embodiment" means that a particular element, feature, structure, or characteristic described in connection with the embodiment is included in at least one embodiment. The appearances of the phrase "in one embodiment" in various places in the specification are not necessarily referring to the same embodiment.

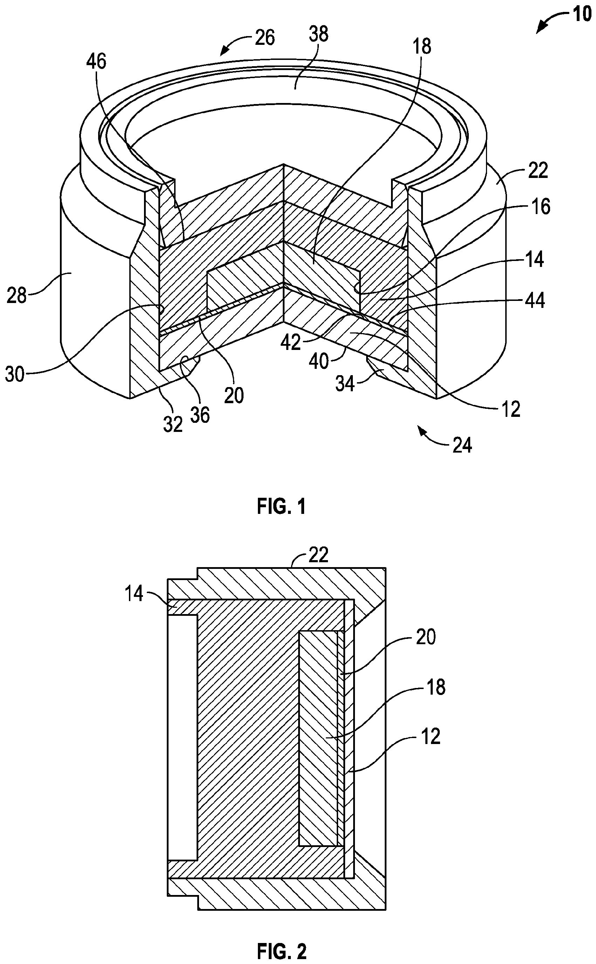

Referring now to the figures, as shown in Figure 1 is an example of a radiation source device 10 for emitting $\gamma$-ray photons, X-ray photons, and fluorescence X-ray photons in a predetermined direction relative to the radiation source device 10 at several energies depending on the radioisotope contained within the radiation source device 10. The radiation source device 10 may be used to measure phase fractions of a multiphase fluid circulating in hydrocarbon exploitation pipes. The fluorescence X-rays generated by the radiation source device 10 may shorten the measurement time for a given phase fraction accuracy or may provide higher phase fraction accuracy for a given measurement of time. The multiphase fluid causes attenuations in the $\gamma$-ray photons, X-ray photons, and fluorescence X-ray photons emitted by the radiation source device 10 which may be employed to calculate the composition of the multiphase fluid. As shown in Figure 1, in one embodiment, the radiation source device 10 has a radiolucent window portion 12 configured to allow $\gamma$-ray photons, X-ray photons, and fluorescence X-ray photons to be emitted in a predetermined direction, a shielding portion 14 having a window portion cavity 16 therein, a radioactive material 18 positioned within the window portion cavity 16 of the shielding portion 14, and a fluorescent material 20 positioned between the radioactive material 18 and the radiolucent window portion 12. The radiation source device 10 also includes a capsule portion 22 housing the radiolucent window portion 12, the shielding portion 14, the radioactive material 18, and the fluorescent material 20. The radiolucent window portion 12 extends across the window portion cavity 16. The radioactive material 18 emits photons through the window portion cavity 16, the fluorescent material 20 and the radiolucent window portion 12. The fluorescent material 20 receives the photons from the radioactive material 18 and generates X-fluorescence photons. In an embodiment where the radioactive material 18 is $^{133}$Ba, the X-fluorescence photons generated have an energy level less than 32 keV and between 15 keV and 25 keV. Although the capsule portion 22 of the radiation source device 10 is depicted as being cylindrical in shape, it will be understood by one skilled in the art that the radiation source device 10 may be sized and shaped in any manner such as cubical, pyramidal, spherical, or the like so long as the radiation source device 10 is capable of emitting photons in a predetermined direction relative to the radiation source device 10.

The capsule portion 22 may be made of a radiopaque material such as stainless steel, nickel-copper alloys, such as Monel® metals, and other suitable radiopaque materials. The capsule portion 22 may be provided with a first side 24, a second side 26, an exterior portion 28 extending between the first side 24 and the second side 26, and an interior portion 30 extending between the first side 24 and the second side 26 opposite the exterior portion 28. The first side 24 may form an open front 32 of the capsule portion 22. The open front 32 may be provided with an outer rim 34 with an inner seating rim 36. The exterior portion 28 may form an outer cylindrical surface of the capsule portion 22 having a diameter of between 3 mm and 10 mm and a height between 3 mm and 10 mm. The interior portion 30 may form a cylindrical inner surface that defines a generally cylindrical space therein. The inner seating rim 36 may project inwardly within the interior portion 30 and may serve to support or contact the radiolucent window portion 12. The interior portion 30 may serve to encapsulate the shielding portion 14. The window portion cavity 16 of the shielding portion 14 may be sized and shaped to receive the radioactive material 18. The shielding portion 14 with its carried radioactive material 18 may be inserted into the capsule portion 22 and seated against the radiolucent window portion 12 such that radiation emitted from the radioactive material 18 emanates from the radiolucent window portion 12, but not from other directions of the radiation source device 10. The shielding portion 14 may be affixed to the capsule portion 22, e.g., by fusion welding. In one embodiment, the shielding portion 14 is affixed to the capsule portion 22 via a cap 38 fitting within a portion of the interior portion 30 of the capsule portion 22, opposite the radiolucent window portion 12, and welded to the capsule portion 22.

The radiolucent window portion 12 is provided with a first side 40 and a second side 42 opposite the first side 40. The radiolucent window portion 12 may be sized and shaped such that the first side 40 of the radiolucent window portion 12 may be positioned against the inner seating rim 36 within the interior portion 30 of the capsule portion 22. The radiolucent window portion 12 may be affixed in place, e.g., by brazing, adhesive, or other suitable mechanisms. The radiolucent window portion 12 may be formed of radiolucent material such as beryllium or fiber reinforced polymers, for example.

The shielding portion 14 may be provided with a first side 44 and a second side 46 opposite the first side 44, with the window portion cavity 16 defined within the first side 44 of the shielding portion 14. The shielding portion 14 may be sized and shaped to fit inside the interior portion 30 of the capsule portion 22. The shielding portion 14 may be positioned within the interior portion 30 such that the first side 44 of the shielding portion 14 may be adjacent to the second side 42 of the radiolucent window portion 12 and so that the radioactive material 18 seated within the window portion cavity 16 may be adjacent to the radiolucent window portion 12. The shielding portion 14 may be constructed from a radiopaque material, such as stainless steel, zirconium, molybdenum, palladium, or silver, nickel-copper alloys, such as Monel®, and the like.

The radioactive material 18 may be configured to fit within the window portion cavity 16 of the shielding portion 14 and adjacent to the radiolucent window portion 12. In one embodiment, the radioactive material 18 is made of a $^{133}$Ba-based ceramic matrix to generate photons having energy levels of 32 keV and 81 keV, where the 32 keV energy level is made by 31 keV and 35 keV X-rays naturally emitted by the $^{133}$Ba radioisotope. The radioactive material 18 may also be formed from $^{109}$Cd, $^{153}$Gd, $^{139}$Ce, $^{152}$Eu, or other suitable radioactive materials. In some embodiments, the radioactive material 18 may be formed from radioactive materials having a single $\gamma$-ray emissions with energy between 40 and 100 keV, such as $^{241}$Am, for example and in this embodiment two different fluorescent materials 20 can be used to generate two different levels of X-ray fluorescent photons to provide three different levels of photon emissions.

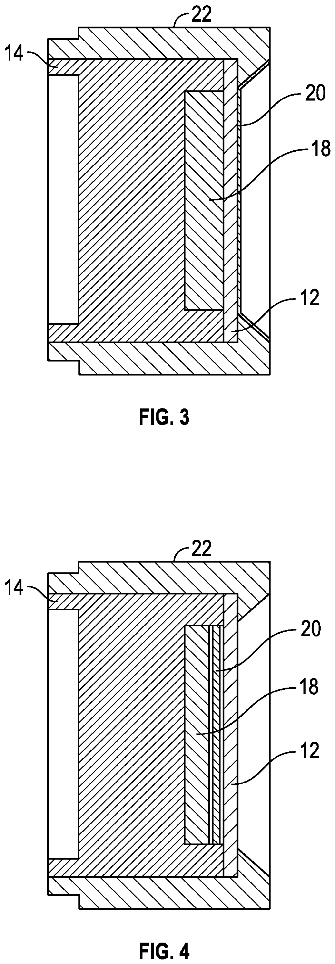

The fluorescent material 20 is positioned between the radioactive material 18 and the radiolucent window portion 12. The fluorescent material 20 may be composed of a metallic material and may be selected from a group consisting of zirconium, molybdenum, palladium, and silver. The fluorescent material 20 may have a thickness in a range from 40 $\mu$m to 200 $\mu$m. In one embodiment, as shown in Figure 2, the fluorescent material 20 may be implemented as a coating applied to the radioactive material 18 or by insertion of a metal foil between the radioactive material 18 and radiolucent window portion 12. In another embodiment, as shown in Figure 3, the fluorescent material 20 may be implemented as a coating applied to the radiolucent window portion 12. In yet another embodiment, as shown in Figure 4, the fluorescent material 20 may be implemented as an independent unit separate from and not attached to the radioactive material 18 and the radiolucent window portion 12 and positioned between the radioactive material 18 and the radiolucent window portion 12. In combination with the radioactive material 18, the fluorescent material 20 may produce a low energy photon beam of between 15 keV and 25 keV which may be detected and interpreted to reduce the statistical uncertainties discussed above.

When assembled within the radiation source device 10 the photons with several energy levels emitted from the radioactive material 18 are received by the fluorescent material 20 and cause the fluorescent material 20 to generate extra photons. In one embodiment, where the radioactive material 18 is $^{133}$Ba, the extra (or X-fluorescence) photons generated by the fluorescent material 20 have an energy level less than 32 keV and are within a range between 15 keV and 25 keV.

Referring now to Figure 2, therein shown is an embodiment, as referenced above, of the radiation source device 10 in which the fluorescent material 20 is a coating applied to the radioactive material 18. The fluorescent material 20 may be applied and attached to the radioactive material 18 as a foil or by vapor deposition of a thin coating onto the radioactive material 18. For example, the fluorescent material 20 may be a thin metallic coating applied by vapor deposition. The thin metallic coating may be chosen from the group consisting of zirconium, molybdenum, palladium and silver, for instance. Although the fluorescent material 20 is described as being deposited as a foil or by vapor deposition, it will be understood by one skilled in the art that the fluorescent material 20 may be applied by other suitable methods.

Referring now to Figure 3, therein shown is an embodiment, as referenced above, of the radiation source device 10 in which the fluorescent material 20 is a coating applied to the radiolucent window portion 12. The fluorescent material 20 may be applied, as described above, as a foil or by vapor deposition of a thin coating onto the radiolucent window portion 12, or may be applied by any other suitable methods. As shown in Figure 3, the fluorescent material 20 may be applied after the radiolucent window portion 12 has been inserted into the capsule portion 22, thereby applying a portion of the fluorescent material 20 to the first side 24 of the capsule portion 22. However, it will be understood by one skilled in the art that the fluorescent material20 may be applied to the radiolucent window portion 12 prior to insertion into the capsule portion 22.

Referring now to Figure 4, shown therein is an embodiment, as referenced above, of the radiation source device 10 in which the fluorescent material 20 is an independent unit separate from and not attached to the radioactive material 18 and the radiolucent window portion 12. As shown in Figure 4, the fluorescent material 20 is positioned between the radioactive material 18 and the radiolucent window portion 12 without being applied as a coating to either the radioactive material 18 or the radiolucent window portion 12. In one embodiment, the fluorescent material 20 may be positioned between the radiolucent window portion 12 and the radioactive material 18. In another embodiment, the fluorescent material 20 may be positioned within a void defined by the radioactive material 18 and the radiolucent window portion 12. In another embodiment, the fluorescent material 20 may be positioned adjacent to an opposing side of the radiolucent window portion 12 such that the radiolucent window portion 12 is positioned against the radioactive material 18 and the fluorescent material 20 is positioned on a side of the radiolucent window portion 12 opposite the radioactive material 18. In this embodiment, for instance, the fluorescent material 20 may be initially placed against the inner seating rim 36 of the capsule portion 22 with the radiolucent window portion 12 being inserted into the capsule portion 22 after the fluorescent material 20 has been positioned and to secure the fluorescent material 20 in place.

The radiation source device 10 may be used in applications such as, for example, measuring devices, multiphase flow meters, phase fraction measuring devices, equipment calibration operations, thickness and density devices, and other applications. Although a few applications are mentioned or discussed at length in the present disclosure, one skilled in the art will understand that the radiation source device 10 may be suitable for use in applications not specifically referenced.

The radiation source device 10 may be used within a measuring device, in one embodiment characterized as a phase fraction measuring device, a multiphase flow meter, or other measurement devices. In general, the measuring device, when characterized as a phase fraction measuring device, may be provided with a fluid passage tube having a first end, a second end, and a cavity extending between the first end and the second end, the radiation source device 10 capable of generating first photons from the radioactive material 18 and second photons from the fluorescent material 20, a photon detector, and a computer. The photon detector may receive the first and second photons passing across the cavity and generate photon signals indicative of a number and energy levels of the first and second photons. The computer may receive the photon signals and calculate the phase fractions of the multiphase fluid with information obtained from the photon signals.

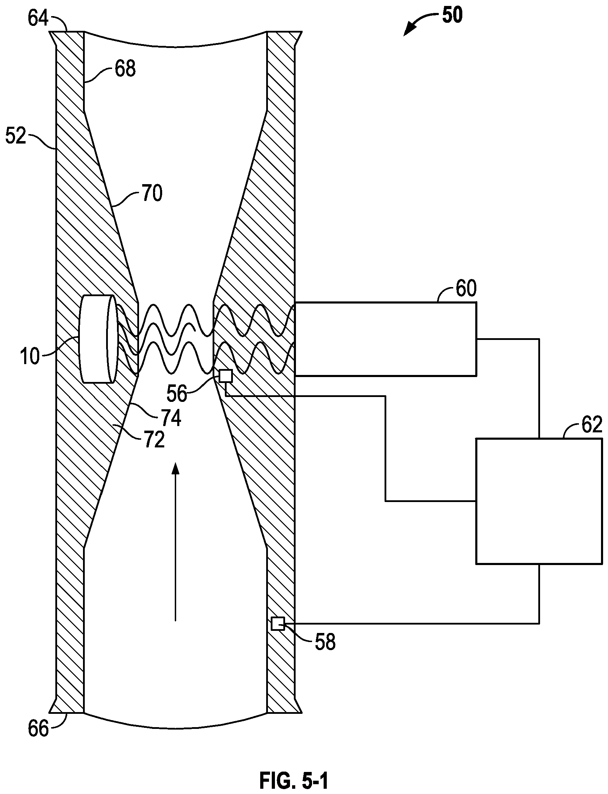

Through photons at (at least) two energy levels, (at least) three phase fractions can be calculated, thus indicating the fluid composition. In order to measure flow rates of each phase of the multiphase fluid, the total flow rate should be known. Thus, when the measuring device 50 includes components to measure the total flow rate, the measuring device 50 can be characterized as a multiphase flow meter. Referring now to Figure 5-1, shown therein is one embodiment of the measuring device 50 characterized as a multiphase flow meter 50. The multiphase flow meter 50 is provided with a venturi tube 52, the radiation source device 10 positioned and configured to emit photons through the venturi tube 52 which interact with the multiphase fluid traveling through the venturi tube 52, a first pressure sensor 56 positioned to sense a first pressure within the venturi tube 52, a second pressure sensor 58 positioned to sense a second pressure within the venturi tube 52, and a photon detector 60 receiving photons passing across the venturi tube 52. The first pressure sensor 56, the second pressure sensor 58, and the photon detector 60 may be coupled to a computer system 62 having a processor such that signals generated by the first pressure sensor 56, the second pressure sensor 58, and the photon detector 60 may be transmitted to the computer system 62 for analysis. Although the multiphase flow meter 50 is shown having a venturi tube 52, other embodiments of multiphase flow meter may not use venturi tubes but rather cause a multiphase fluid to pass through a pipe or other container in order to be analyzed.

The venturi tube 52 is provided with a first end 64, a second end 66, and a cavity 68 extending between the first end 64 and the second end 66. The first end 64 and the second end 66 may be configured to connect to piping through which a fluid flow sampled from a downhole formation is passed. For example, the first and second ends 64 and 66 may be threaded, flanged and configured to accept bolts, or may have clamps configured to connect to piping, such that the venturi tube 52 may be installed in the fluid flow and allow the sample to pass through the venturi tube 52. The venturi tube 52 has a protrusion 72 adjacent to an inner surface 70 to define a venturi tube throat 74. The venturi tube throat 74 causes a pressure drop when a multiphase fluid, such as a combination of liquid and gas, flows through the venturi tube 52 and thereby through the venturi tube throat 74. The venturi tube 52 may have a ratio between an interior diameter of the venturi tube throat 74 and the interior diameter of the venturi tube 52 of 0.5. The venturi tube 52 may be constructed as a tube of 38 mm, 80 mm, 130 mm, or any other suitable diameter. In one embodiment, the venturi tube 52 may be constructed of radiopaque material, having window portions constructed from radiolucent materials, such that the radiation source device 10 may be positioned to emit photons through a first window portion at the venturi tube throat 74 so that the photons pass through the first window portion and a second window portion, opposite and facing the first window portion relative to the venturi tube 52, to be received by the photon detector 60.

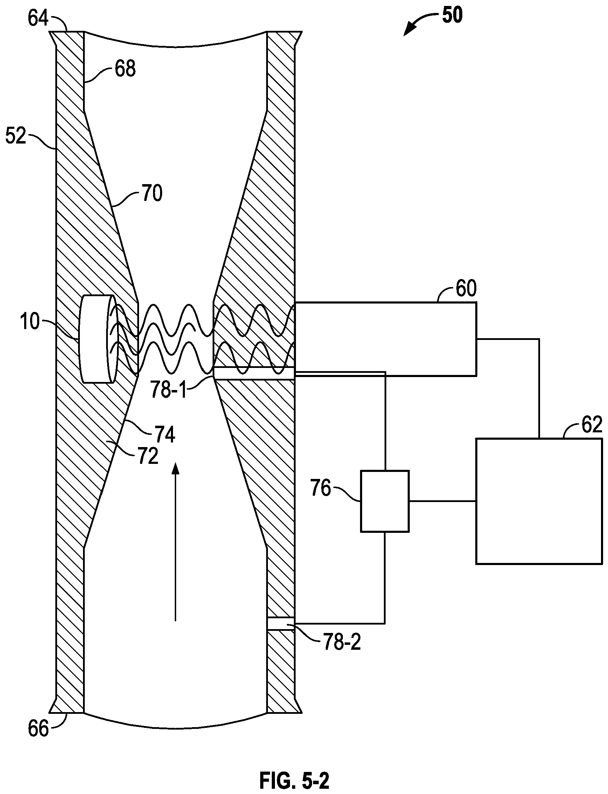

In one embodiment, the radiation source device 10 may be positioned and configured to emit photons across the cavity 68 at the venturi tube throat 74. However, the radiation source device 10 may be positioned at varying places along the venturi tube 52, including outside of the venturi tube throat 74. In one embodiment, where the radioactive material 18 is $^{133}Ba$, the photons have energy levels greater than or equal to 32 keV and extra photons generated by the fluorescent material 20 have an energy level less than 32 keV. As shown in Figure 5, the radiation source device 10 may be positioned within the venturi tube 52 in a recess adjacent to the venturi tube throat 74.

The first pressure sensor 56 may sense a first pressure within the venturi tube throat 74 and generate first pressure signals indicative of the first pressure. The first pressure sensor 56 may transmit the first pressure signals to the computer system 62 for use in determining the multiphase flow of a fluid traveling through the venturi tube 52. The first pressure sensor 56 may be implemented as any type of pressure sensor capable of sensing pressure within the venturi tube throat 74 and transmitting the first pressure signals indicative of that pressure to the computer system 62.

The second pressure sensor 58 may sense a second pressure outside of the venturi tube throat 74 and generate second pressure signals indicative of the second pressure. The second pressure sensor 58 may transmit the second pressure signals to the computer system 62 for use in determining the multiphase flow of the fluid traveling through the venturi tube 52. The second pressure sensor 58 may be implemented as any suitable pressure sensor capable of sensing pressure within venturi tube 52 outside of the venturi tube throat 74 and transmitting the second pressure signals indicative of that pressure to the computer system 62.

In one embodiment, as shown in Figure 5-2, the first and second pressure sensors 56 and 58 may be implemented as a differential pressure sensor 76. In this embodiment, the differential pressure sensor 76 may be connected to the venturi tube 52 adjacent to a first pressure measurement opening 78-1 and a second pressure measurement opening 78-2 and may be configured to measure a pressure difference between the first and second pressure measurement openings 78-1 and 78-2. The first pressure measurement opening 78-1 is positioned at the venturi tube throat 74 and the second pressure measurement opening 78-2 is positioned prior to or upstream of the venturi tube throat 74. In relation to Figure 5-1, the first pressure measurement opening 78-1 may substitute and be located at the position of the first pressure sensor 56 while the second pressure measurement opening 78-2 may substitute and be located at the position of the second pressure sensor 58. The first and second pressure measurement openings 78-1 and 78-2 may be connected to the differential pressure sensor 76 such that the differential pressure sensor may detect a differential pressure, indicative of the change in pressure between the first pressure measurement opening 78-1 and the second pressure measurement opening 78-2, within the fluid traveling through the venturi tube 52 located at or near the venturi tube throat 74 and generate at least one pressure signal indicative of the differential pressure.

Although shown in Figure 5-1 and 5-2 as being provided with a venturi tube 52 and a venturi tube throat 74, some embodiments of a phase fraction measuring device or a multiphase flow meter may not be provided with a venturi tube 52 or a venturi tube throat 74. For example, in one embodiment a measuring device, characterized as a phase fraction measuring device, may be provided with a fluid passage tube, such as a pipe or other container, configured to receive and allow passage of a multiphase fluid to be analyzed by the radiation source device 10, the photon detector 60, and the computer system 62. Further, a measuring device, can be characterized as a multiphase flow meter and in such case the multiphase flow meter has a device to measuring the flow of the multiphase fluid, such as the differential pressure sensor 76 in Figure 5-2, or the first and second pressure sensors 56 and 58 in Figure 5-1, in addition to the fluid passage tube, the radiation source device 10, the photon detector 60, and the computer system 62. In one embodiment, the multiphase flow meter may be provided with a coriolis or other flow meter rather than the differential pressure sensor 76, to sense the flow rate of the multiphase fluid through the fluid passage tube. Although the phase fraction measuring device may be described as employing venturi tubes, pipes, or containers, and the multiphase flow meter may additionally be described as employing pressure sensors, differential pressure sensors, or coriolis flow meters, it will be understood by one skilled in the art that the multiphase flow meter and the phase fraction measuring device may be constructed in a number of different ways while remaining within the scope of the present disclosure.

The photon detector 60 may receive the photons passing across the cavity 68 at the venturi tube throat 74 and generate photon signals indicative of the number and energy level of the photons. The photon detector 60 may transmit the photon signals to the computer system 62 for use in determining the multiphase flow of the fluid traveling through the venturi tube 52. The photon detector 60 may be positioned opposite the radiation source device 10 and may be at least partially supported by a recess within the venturi tube 52. The photon detector 60 may be implemented as any suitable photon detector capable of generating electrical signals indicative of photons that are received from the radiation source device 10. For example, the photon detector 60 can be a scintillator detector, capable of detecting γ-rays and X-rays. The scintillator may be composed of YAP(Ce), NaI(Tl), or $CeBr_3$, for example. The detector may be completed with a photomultiplier tube and a power supply.

The computer system 62 may be provided with a processor, a non-transitory computer readable medium, processor executable instructions stored on the non-transitory computer readable medium, an input device, an output device, and a communications device. The processor may be implemented as a single processor or multiple processors working together or independently to execute the processor executable instructions. The processor is coupled to the non-transitory computer readable medium which may be implemented as RAM, ROM, flash memory or the like, and may take the form of a magnetic device, optical device, or the like. The input device may transmit data to the processor and may be implemented as a keyboard, a mouse, a touch-screen, a camera, a cellular phone, a tablet, a smart phone, a PDA, a microphone, a network adapter, cable adapter such as a USB port, a scanner, and combinations thereof. The output device transmits information from the processor to a user and may be implemented as a server, a computer monitor, a cell phone, a tablet, a speaker, a website, a PDA, a fax, a printer, a projector, a laptop monitor, and combinations thereof. The network communications device may facilitate communications between a network and the processor. Stored on the non-transitory computer readable medium, the processor executable instructions, when executed by the processor, may cause the processor to receive the at least one pressure signal and photon signals from the first and second pressure sensors 56 and 58 and the photon detector 60 and calculate a composition of the multiphase fluid traveling through the venturi tube 52. The composition of the multiphase fluid, i.e. the phase fractions, may be given by the photon signals from the photon detector 60. The flow rate of each individual phase of the multiphase fluid may be calculated using a pressure difference between the first pressure signals and the second pressure signals, generating a total flow rate, and the photon signals. In the embodiment having the differential pressure sensor 76, the flow rate of the individual phases of the multiphase fluid may be calculated using the at least one pressure signal indicative of the differential pressure and the photon signal from the photon detector.

A method for using a measuring device 50, generally, may include installing the phase fraction measuring device 50 in a fluid flow sampled from a downhole formation. The phase fraction measuring device 50 may have the fluid passage tube 52 with a cavity, a radiation source device 10 with the radioactive material 18 and the fluorescent material 20. The radioactive material 18 generates first photons and the fluorescent material 20 receives the first photons from the radioactive material and generates second photons. The radiation source device 10 is positioned and configured to emit the first and second photons across the cavity to interact with a multiphase fluid passing through the fluid passage tube. The photon detector 60 may receive the photons passing across the cavity and interacting with the multiphase fluid passing through the fluid passage tube 52. After installation of the radiation source device 10, photon signals may be generated via the photon detector 60 indicative of a number and energy levels of the first and second photons. Data indicative of the photon signal may be logged onto a non-transitory computer readable medium.

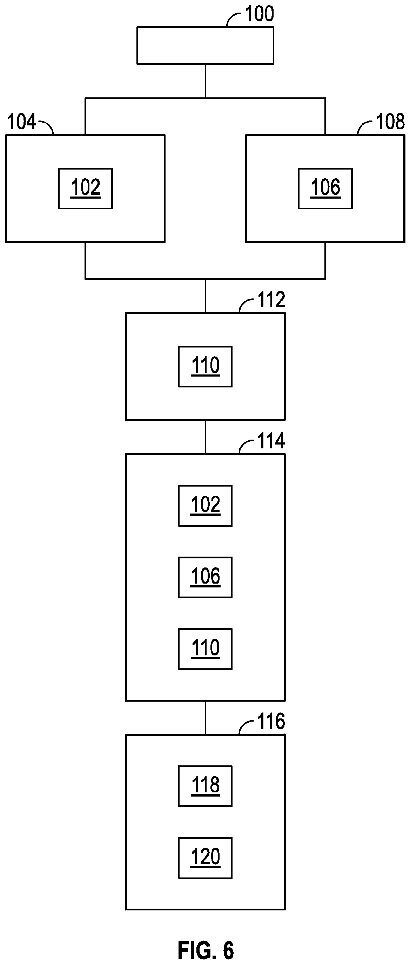

Referring now to Figure 6, shown therein is one embodiment of a method for installing and using the measuring device 50 of Figure 5. The measuring device 50 may be installed at block 100. The measuring device 50 may be installed in a fluid flow sampled from a downhole formation. For example, the measuring device 50 may be installed between two sections of pipe through which a fluid flow sampled from a downhole formation is directed. The measuring device 50 may be connected using threaded connections at the first and second ends 64 and 66 or any other suitable mechanism to enable passage of the fluid flow through the measuring device 50. Once installed, the measuring device 50 may generate first pressure signals 102 via the first pressure sensor 56 indicative of a first pressure of the multiphase fluid passing through the venturi tube 52 at the venturi tube throat 74, at block 104. Block 104 may also represent at least one pressure signal 102 indicative of the differential pressure generated by the differential pressure sensor 76, as shown in Figure 5-2. The measuring device 50 may also generate second pressure signals 106 via the second pressure sensor 58 indicative of the second pressure of the multiphase fluid passing through the venturi tube 52 prior to or after the venturi tube throat 74. The measuring device 50 may generate photon signals 110 at block 112 via the photon detector 60. The photon signals 110 are indicative of the number and the energy levels of the photons emitted by the radiation source device 10 passing through the venturi tube 52 and the multiphase fluid at the venturi tube throat 74.

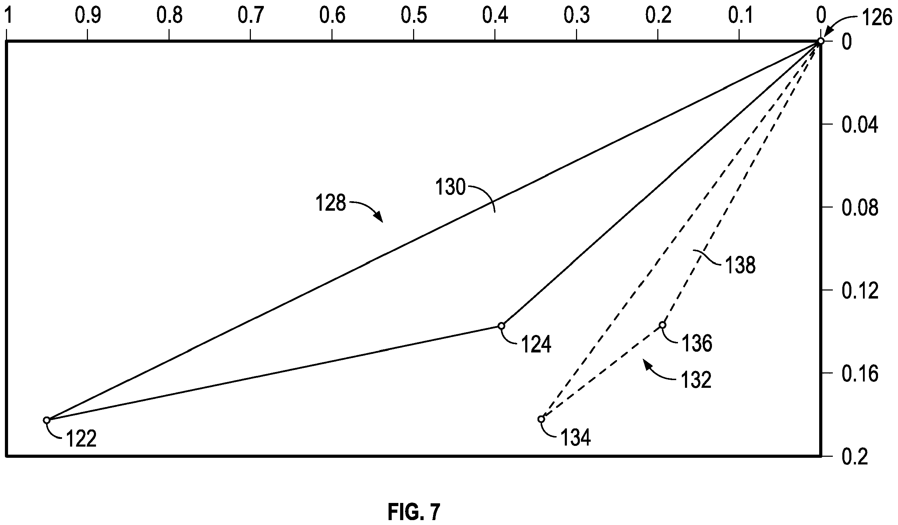

At block 114, the measuring device 50 logs data indicative of the first pressure signal, the second pressure signal, and the photon signal 102, 106, and 110 onto the non-transitory computer readable medium of the computer system 62. As will be described in further detail below in relation to Figure 7, the computer system 62 may calculate the single phase flow rates of the multiphase fluid traveling through the venturi tube 52 at block 116. The computer system 62 may calculate the total flow rate of the multiphase fluid by calculating a pressure difference 118 between the first pressure signals 102 and the second pressure signals 106 generating a total flow rate for the multiphase fluid. The computer system 62 may then calculate the single phase flow rates 120 within the multiphase fluid from the photon signals 110 and the pressure difference 118. The pressure difference 118 may also be taken from the differential pressure of the at least one pressure signal 102 generated by the differential pressure sensor 76.

Installing the measuring device 50 may further involve calibrating the measuring device 50. Calculating the phase fraction of the multiphase fluid may be based on Beer-Lambert law using equation 1: $n_E = n_{E,0} \exp(-\Sigma_{i=w,o,g} \alpha_i \lambda_{E,i} d)$. In equation 1, $n_E$ *is* a number of photons per second with energy $E$ averaged over a time interval $t$, $n_{E,0}$ is a number of photons per second with energy $E$ detected when the venturi tube 52 is empty, $\lambda_{E,i}$ are linear attenuation coefficients, in $cm^{-1}$, for the energy $E$ for phases (water, oil, gas) of the multiphase fluid, d is a photon beam propagation distance through the multiphase fluid. A calibration process may be used to determine the linear attenuation coefficients $\lambda_{E,i}$. The calibration process may be performed prior to or after taking samples from the fluid flow sampled from the downhole formation.

The calibration process may be performed by filling the venturi tube 52 with 100% water, generating the photon signal 110 for water. The venturi tube 52 may then be filled with 100% oil and the phase fraction measuring device 50 may then generate the photon signal 110 for oil. The venturi tube 52 may then be filled with 100% gas and the measuring device 50 may then generate the photon signal 110 for gas. The computer system 62 may then be used to generate a graph, represented by Figure 7, using the data indicative of the photon signals 102 for the water, oil, and gas, respectively.

The phase fractions 120 of the multiphase fluid traveling through the phase fraction measuring device 50 is calculated using the photon signals 110. The low energy fluorescent emission below 25 keV, in addition to the natural emissions of 32 keV and 81 keV, in the embodiment using $^{133}Ba$, enable an additional low energy photon beam. The additional low energy photon beam is used in conjunction with the natural emissions to calculate the phase fractions of the multiphase fluid with a shorter measurement time for a predetermined accuracy or with increased accuracy for the same measurement time. Entering the three energy levels (<25 keV, 32 keV, and 81 keV for $^{133}Ba$), into equation 1 may generate equation 2:

$$\begin{pmatrix} \lambda_{E_{Xfluo},mix} \\ \lambda_{32,mix} \\ \lambda_{81,mix} \\ 1 \end{pmatrix} = \underbrace{\begin{pmatrix} \lambda_{E_{Xfluo},w} & \lambda_{E_{Xfluo},o} & \lambda_{E_{Xfluo},g} \\ \lambda_{32,w} & \lambda_{32,o} & \lambda_{32,g} \\ \lambda_{81,w} & \lambda_{81,o} & \lambda_{81,g} \\ 1 & 1 & 1 \end{pmatrix}}_{M} \begin{pmatrix} \alpha_w \\ \alpha_o \\ \alpha_g \end{pmatrix},$$

which may be solved to calculate the phase fractions. In equation 2, $\lambda_{E_{Xfluo},mix}$ represents measured linear coefficients at the energy level $E_{Xfluo}$ below 25 keV for the mixture, $\lambda_{32,mix}$ represents measured linear coefficients at the low energy level 32 keV, $\lambda_{81,mix}$ represents measured linear coefficients at the high energy 81 keV in the embodiment using $^{133}Ba$, and $\alpha_i$ represents the phase fractions, with $i$ representing either water, oil, or gas. By considering the Beer-Lambert law of equation 1 for three energy levels, the linear system becomes over-determined for three unknowns ($\alpha_i$) and four available equations. The computer system 62 may solve equation 2 using a weighted linear least squares method to give a unique solution, for example.

As shown in Figure 7, the computer system 62 may calculate the linear attenuation coefficients indicative of the attenuation of the $\gamma$- and X-rays passing through the water, oil, and gas. The graph, representative of the linear attenuation coefficients, has an x axis $\lambda_{LE}$ plotting points indicative of the linear attenuation coefficient values at low energy and a y axis $\lambda_{HE}$ plotting points indicative of linear attenuation coefficient values at high energy. A linear attenuation coefficient for water 134 indicative of the photon signals 110 passing through 100% water, a linear attenuation coefficient for oil 136 indicative of the photon signals 110 passing through 100% oil, and a linear attenuation coefficient for gas 126 indicative of the photon signals 110 passing through 100% gas are calculated by the computer system 62 and may be plotted on the graph of Figure 7 forming a first attenuation triangle 132 with an area 138. The larger the area 138 of the triangle 132, the smaller the phase fraction uncertainties for the same acquisition time, or conversely, shorter acquisition time for the same phase fraction accuracy. This triangle has a linear attenuation coefficient for water 134 indicative of the photon signals 110 passing through 100% water, a linear coefficient for oil 136 indicative of the photon signals 110 passing through 100% oil, and the linear coefficient for gas 126 indicative of the photon signals 110 passing through 100% gas. The attenuation triangle 132 forms an area 138. The attenuation triangle 132 plots attenuation points for [32; 81] keV, where 32 keV is the low energy and 81 keV is the high energy, in the embodiment using $^{133}Ba$. The area 138 corresponds to a scenario with photons emitted by $^{133}Ba$ without X-fluorescence and the possible phase fraction combinations are distributed across the area 138. A second attenuation triangle 128 is representative of plots of attenuations points for [$E_{Xfluo}$; 81] keV where $E_{Xfluo}$ is the low energy, indicative of the fluorescence X-rays generated by the fluorescence material of the radiation source device 10, where $E_{Xfluo}$ is below 25 keV, and 81 keV is the high energy, in the embodiment using $^{133}Ba$. The area 130 corresponds to a scenario with photons emitted by $^{133}Ba$ with X-fluorescence and the possible phase fraction combinations are distributed across the area 130, larger than 138.

For example, let $M$ be the attenuation matrix. A system of equation 2 has a unique solution when det($M$) # 0, e.g., rows are linearly independent. It may be noted that the attenuation triangles areas 130 and 138, of Figure 7, correspond to det($M$). By assuming a perfect calibration, statistical uncertainties solely affect the left-hand column matrix.

In an inversion process for solving the system, a larger det($M$) may correlate to a smaller uncertainty amplification.

The same technique described above can be used with different couples of low and high energies by choosing different radioisotopes from $^{133}$Ba, such as $^{109}$Cd, $^{153}$Gd, $^{139}$Ce, and $^{152}$Eu, for example. In another example, radioisotopes emitting a single $\gamma$-ray with energy between 40 keV and 100 keV can be used, such as $^{241}$Am. In order to generate two extra energies for the attenuation matrix through X-ray fluorescence, the fluorescent material may be formed by a two-layer assembly of sheets or by an alloy of two metals, such that each of the metals within the alloy produce a differing fluorescent energy.

Although the preceding description has been described herein with reference to particular means, materials and embodiments, it is not intended to be limited to the particulars disclosed herein; rather, it extends to all functionally equivalent structures, methods, and uses, such as are within the scope of the appended claims.

## Claims

**1.** A radiation source device (10), comprising:

a radiolucent window portion (12);
a shielding portion (14) having a window portion cavity (16) therein, the radiolucent window portion (12) being located in front of and extending across the window portion cavity (16);
a radioactive material (18) positioned within the window portion cavity (16) of the shield portion so as to emit first photons through the window portion cavity and the radiolucent window portion;
a fluorescent material (20) receiving the first photons from the radioactive material and generating second photons, the fluorescent material being positioned at one of: (i) between the radioactive material and the radiolucent window portion; and (ii) adjacent an opposite side of the radiolucent window portion from the radioactive material; and
a capsule (22) housing the radiolucent window portion, the shielding portion, the radioactive material and the fluorescent material together,
**characterized in that**
the radioactive material (18) is formed from radioactive material having single $\gamma$-ray emissions with energy between 40 and 100 keV and the fluorescent material (20) comprises two different fluorescent materials (20) and/or includes a two-layer assembly of sheets of two metals or an alloy of two metals, to provide three different energy levels of photon emissions; or
the radioactive material (18) includes $^{133}$Ba, $^{109}$Cd, $^{153}$Gd, $^{139}$Ce, or $^{152}$Eu, and wherein photons emitted by the radiation source device (10) are at a first energy level, at a second energy level, and at a third energy level, wherein the second photons generated by the fluorescent material (20) have an energy level between 15 keV and 25 keV.

**2.** The radiation source device of claim 1, wherein the fluorescent material (20) is a metallic material.

**3.** The radiation source device of claim 2, wherein the fluorescent material (20) is selected from a group consisting of zirconium, molybdenum, palladium and silver.

**4.** The radiation source device of any of the preceding claims, wherein the fluorescent material (20) is a coating applied to the radioactive material or the radiolucent window portion.

**5.** The radiation source device of any of claims 1 to 3, wherein when the fluorescent material (20) is positioned between the radioactive material and the radiolucent window portion, the fluorescent material is an independent unit separate from the radioactive material and the radiolucent window portion.

**6.** A measuring device (50), comprising:

a fluid passage tube (52) having a first end (64), a second end (66) and a cavity (68) extending between the first end and the second end;
a radiation source device (10) according to any one of the preceding claims, wherein the radiation source device is positioned and configured to emit the first and second photons across the cavity (68) to interact with multiphase fluid within the cavity (68);
a photon detector (60) receiving the first and second photons passing across the cavity interacting with the multiphase fluid passing through the fluid passage tube and generating photon signals indicative of a number and energy levels of the first and second photons; and
a computer (62) receiving the photon signals and calculating the phase fractions of the multiphase fluid with information obtained from the photon signals.

**7.** The measuring device of claim 6, wherein the fluorescent material (20) is a metallic material.

**8.** The measuring device of claim 7, wherein the fluorescent material (20) is selected from a group consisting of zirconium, molybdenum, palladium and silver.

**9.** The measuring device of any of claims 6 to 8, wherein the fluorescent material (20) is a coating applied to the radioactive material.

**10.** The measuring device of any of claims 6 to 8, wherein the fluorescent material (20) is a coating applied to the radiolucent window portion.

**11.** The measuring device of any of claims 6 to 8, wherein when the fluorescent material (20) is positioned between the radioactive material and the radiolucent window portion, the fluorescent material is an independent unit separate from the radioactive material and the radiolucent window portion and positioned therebetween.

**12.** A method, comprising:

installing a measuring device (50) in accordance with any of claims 6 to 11 in a fluid flow sampled from a downhole formation;
generating photon signals via the photon detector (60) indicative of a number and energy levels of the first and second photons; and
logging data indicative of the photon signal onto a non-transitory computer readable medium.

**Patentansprüche**

**1.** Strahlungsquellenvorrichtung (10), umfassend:

einen strahlendurchlässigen Fensterabschnitt (12);
einen Abschirmabschnitt (14) mit einem Fensterabschnitthohlraum (16) darin, wobei sich der strahlendurchlässige Fensterabschnitt (12) vor dem Fensterabschnitthohlraum (16) befindet und über diesen erstreckt;
ein radioaktives Material (18), das innerhalb des Fensterabschnitthohlraums (16) des Abschirmungsabschnitts positioniert ist, um erste Photonen durch den Fensterabschnitthohlraum und den strahlendurchlässigen Fensterabschnitt hindurch zu emittieren;
ein fluoreszierendes Material (20), das die ersten Photonen aus dem radioaktiven Material empfängt und zweite Photonen erzeugt, wobei das fluoreszierende Material an einer Position positioniert ist aus: (i) zwischen dem radioaktiven Material und dem strahlendurchlässigen Fensterabschnitt; und (ii) angrenzend an eine dem radioaktiven Material gegenüberliegende Seite des strahlendurchlässigen Fensterabschnitts; und
eine Kapsel (22), in der der strahlendurchlässige Fensterabschnitt, der Abschirmabschnitt, das radioaktive Material und das fluoreszierende Material zusammen untergebracht sind,
**dadurch gekennzeichnet, dass**
das radioaktive Material (18) ausgebildet ist aus einem radioaktiven Material, das einzelne $\gamma$-Strahlen-Emissionen mit einer Energie zwischen 40 und 100 keV aufweist, und das fluoreszierende Material (20) zwei verschiedene fluoreszierende Materialen (20) umfasst und/oder eine zweischichtige Anordnung von Blechen aus zwei Metallen oder einer Legierung aus zwei Metallen umfasst, um drei verschiedene Energieniveaus von Photonenemissionen bereitzustellen; oder das radioaktive Material (18) $^{133}$Ba, $^{109}$Cd, $^{153}$Gd, $^{139}$Ce oder $^{152}$Eu umfasst, und
wobei sich aus der Strahlungsquellenvorrichtung (10) emittierte Photonen auf einem ersten Energieniveau, auf einem zweiten Energieniveau und auf einem dritten Energieniveau befinden, wobei die vom fluoreszierenden Material (20) erzeugten zweiten Photonen ein Energieniveau zwischen 15 und 25 keV aufweisen.

**2.** Strahlungsquellenvorrichtung nach Anspruch 1, wobei das fluoreszierende Material (20) ein metallisches Material ist.

**3.** Strahlungsquellenvorrichtung nach Anspruch 2, wobei das fluoreszierende Material (20) aus einer Gruppe bestehend aus Zirkonium, Molybdän, Palladium und Silber ausgewählt ist.

**4.** Strahlungsquellenvorrichtung nach einem der vorhergehenden Ansprüche, wobei das fluoreszierende Material (20) eine auf das radioaktive Material oder den strahlendurchlässigen Fensterabschnitt aufgebrachte Beschichtung ist.

**5.** Strahlungsquellenvorrichtung nach einem der Ansprüche 1 bis 3, wobei, wenn das fluoreszierende Material (20) zwischen das radioaktive Material und den strahlendurchlässigen Fensterabschnitt positioniert ist, das fluoreszierende Material eine vom radioaktiven Material und dem strahlendurchlässigen Fensterabschnitt separate unabhängige Einheit ist.

**6.** Messvorrichtung (50), umfassend:

ein Fluiddurchtrittsrohr (52) mit einem ersten Ende (64), einem zweiten Ende (66) und einem sich zwischen dem ersten Ende und dem zweiten Ende erstreckenden Hohlraum (68);
eine Strahlungsquellenvorrichtung (10) gemäß einem der vorhergehenden Ansprüche, wobei die Strahlungsquellenvorrichtung positioniert und ausgelegt ist, die ersten und zweiten Photonen quer durch den Hohlraum (68) zu emittieren, um mit Mehrphasenfluid innerhalb des Hohlraums (68) zu interagieren;
einen Photonendetektor (60), der die ersten und zweiten Photonen empfängt, die den Hohlraum durchqueren, mit dem durch das Fluiddurchtrittsrohr hindurchtretenden Mehrphasenfluid interagieren und Photonensignale erzeugen, die eine Anzahl und Energieniveaus der ersten und zweiten Photonen anzeigen; und
einen Rechner (62), der die Photonensignale empfängt und die Phasenanteile des Mehrphasenfluids mit aus den Photonensignalen erhaltenen Informationen berechnet.

**7.** Messvorrichtung nach Anspruch 6, wobei das fluoreszierende Material (20) ein metallisches Material ist.

**8.** Messvorrichtung nach Anspruch 7, wobei das fluoreszierende Material (20) aus einer Gruppe bestehend aus Zirkonium, Molybdän, Palladium und Silber ausgewählt ist.

**9.** Messvorrichtung nach einem der Ansprüche 6 bis 8, wobei das fluoreszierende Material (20) ein auf das radioaktive Material aufgebrachte Beschichtung ist.

**10.** Messvorrichtung nach einem der Ansprüche 6 bis 8, wobei das fluoreszierende Material (20) eine auf den strahlendurchlässigen Fensterabschnitt aufgebrachte Beschichtung ist.

**11.** Messvorrichtung nach einem der Ansprüche 6 bis 8, wobei, wenn das fluoreszierende Material (20) zwischen dem radioaktiven Material und dem strahlendurchlässigen Fensterabschnitt positioniert ist, das fluoreszierende Material eine vom radioaktiven Material und dem strahlendurchlässigen Fensterabschnitt separate unabhängige und zwischen diesen positionierte Einheit ist.

**12.** Verfahren, umfassend:

Anbringen einer Messvorrichtung (50) gemäß einem der Ansprüche 6 bis 11 in einem aus einer Bohrlochformation beprobten Fluidstrom;
Erzeugen von Photonensignalen mithilfe des Photonendetektors (60), die eine Anzahl und Energieniveaus der ersten und zweiten Photonen anzeigen; und
Loggen von das Photonensignal anzeigenden Daten auf einem nichtflüchtigen rechnerlesbaren Medium.

## Revendications

**1.** Dispositif de source de rayonnement (10), comprenant :

une partie fenêtre radiotransparente (12) ;
une partie blindage (14) présentant une cavité de la partie fenêtre (16) dans celle-ci, la partie fenêtre radiotransparente (12) étant située à l'avant et s'étendant sur l'étendue de la cavité de la partie fenêtre (16) ;
un matériau radioactif (18) positionné à l'intérieur de la cavité de la partie fenêtre (16) de la partie blindage de façon à émettre des premiers photons à travers la cavité de la partie fenêtre et la partie fenêtre radiotransparente ;
un matériau fluorescent (20) recevant les premiers photons provenant du matériau radioactif et générant des seconds photons, le matériau fluorescent étant positionné soit : (i) entre le matériau radioactif et la partie fenêtre radiotransparente ; et (ii) soit adjacent à un côté opposé de la partie fenêtre radiotransparente à partir du matériau radioactif ;

une capsule (22) hébergeant la partie fenêtre radiotransparente, la partie blindage, le matériau radioactif et le matériau fluorescent **caractérisé en ce que**

le matériau radioactif (18) est constitué de matériau radioactif présentant des émissions de rayon y unique comportant une énergie entre 40 et 100 keV et le matériau fluorescent (20) comprend deux matériaux fluorescent différents (20) et/ou inclut un ensemble à deux couches de feuilles de deux métaux ou d'un alliage de deux métaux, pour fournir trois différents niveaux d'énergie des émissions de photons ;

le matériau radioactif (18) inclut $^{133}$Ba, $^{109}$Cd, $^{153}$Gd, $^{139}$Ce, $^{152}$Eu et dans lequel les photons émis par le dispositif de source de rayonnement (10) sont à un premier niveau d'énergie, à un deuxième niveau d'énergie et à un troisième niveau d'énergie, dans lequel les seconds photons générés par le matériau fluorescent (20) présentent un niveau d'énergie entre 15 keV et 25 keV.

**2.** Dispositif de source de rayonnement selon la revendication 1, dans lequel le matériau fluorescent (20) est un matériau métallique.

**3.** Dispositif de source de rayonnement selon la revendication 2, dans lequel le matériau fluorescent (20) est sélectionné dans un groupe constitué par le zirconium, le molybdène, le palladium et l'argent.

**4.** Dispositif de source de rayonnement selon l'une quelconque des revendications précédentes, dans lequel le matériau fluorescent (20) est un revêtement appliqué au matériau radioactif ou à la partie fenêtre radiotransparente.

**5.** Dispositif de source de rayonnement selon l'une quelconque des revendications 1 à 3, dans lequel lorsque le matériau fluorescent (20) est positionné entre le matériau radioactif et la partie fenêtre radiotransparente, le matériau fluorescent est une unité indépendante distincte du matériau radioactif et de la partie fenêtre radiotransparente.

**6.** Dispositif de mesure (50), comprenant :

un tube de passage de fluide (52) présentant une première extrémité (64), une seconde extrémité (66) et une cavité (68) s'étendant entre la première extrémité et la seconde extrémité ;

un dispositif de source de rayonnement (10) selon l'une quelconque des revendications précédentes, dans lequel ledit dispositif de source de rayonnement est positionné et conçu pour émettre les premiers et les seconds photons sur l'étendue de la cavité (68) pour interagir avec un fluide multiphase à l'intérieur de la cavité (68) ;

un détecteur de photons (60) recevant les premiers et les seconds photons passant sur l'étendue de la cavité interagissant avec le fluide multiphase traversant le tube de passage du fluide et générant des signaux photoniques indiquant un nombre et des niveaux d'énergie des premiers et des seconds photons ; et

un ordinateur (62) recevant les signaux photoniques et calculant les fractions de phase du fluide multiphase à partir d'informations obtenues à partir des signaux photoniques.

**7.** Dispositif de mesure selon la revendication 6, dans lequel le matériau fluorescent (20) est un matériau métallique.

**8.** Dispositif de mesure selon la revendication 6, dans lequel le matériau fluorescent (20) est sélectionné dans un groupe constitué par le zirconium, le molybdène, le palladium et l'argent.

**9.** Dispositif de mesure selon l'une quelconque des revendications 6 à 8, dans lequel le matériau fluorescent (20) est un revêtement appliqué au matériau radioactif.

**10.** Dispositif de mesure selon l'une quelconque des revendications 6 à 8, dans lequel le matériau fluorescent (20) est un revêtement appliqué à la partie fenêtre radiotransparente.

**11.** Dispositif de mesure selon l'une quelconque des revendications 6 à 8, dans lequel le matériau fluorescent (20) est positionné entre le matériau radioactif et la partie fenêtre radiotransparente, le matériau fluorescent est une unité indépendante distincte du matériau radioactif et de la partie fenêtre radiotransparente et positionnée entre celles-ci.

**12.** Procédé, comprenant l'installation d'un dispositif de mesure (50) conformément à l'une quelconque des revendications 6 à 11 dans un écoulement de fluide échantillonné à partir d'une formation de fond de trou ;
la génération des signaux photoniques par l'intermédiaire du détecteur de photons (60) indiquant un nombre et des niveaux d’énergie des premiers et seconds photons ; et
les données d’enregistrement indiquant le signal photonique sur un support lisible par ordinateur non transitoire.

10
26
38
18
46
22
16
14
28
44
42
40
30
34
20
12
32
36
24

FIG. 1

22
14
20
18
12

FIG. 2

22
14
20
18
12

FIG. 3

22
14
20
18
12

FIG. 4

64
50
68
52
70
60
10
56
74
72
62
58
66

FIG. 5-1

50
64
68
52
70
60
10
78-1
74
72
62
76
78-2
66

FIG. 5-2

100
104
102
108
106
112
110
114
102
106
110
116
118
120

FIG. 6

126
0
0.04
0.08
0.12
0.16
0.2
0
0.1
0.2
0.3
0.4
0.5
0.6
0.7
0.8
0.9
1
138
136
132
134
124
130
128
122

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4388530 A
- US 20110278445 A
- US 4450576 A
- DE 3424937
- US 3655984 A